# EUROPEAN PATENT APPLICATION

(11) **EP 1 044 694 A1**
(43) Date of publication of application: **18.10.2000**
(21) Application number: 99940650.7
(22) Date of filing: 03.09.1999
(51) Int. Cl.: A61L 27/00, C08L 1/00

(54) **POLYMERIC MATERIAL FOR ARTIFICIAL BONE**

(30) Priority: 06.09.1998 JP 26901098
(71) Applicant: Japan Science and Technology Corporation, Kawaguchi-shi, Saitama 332-0012 (JP)
(72) Inventor: MIYAMOTO, Takeaki, Nagaokakyo-shi, Kyoto 617-0845 (JP); KOKUBO, Tadashi, Nagaokakyo-shi, Kyoto 617-0841 (JP)
(74) Representative: Calamita, Roberto
(86) International application number: JP9904791
(87) International publication number: WO0013716

(57) **Abstract**

A novel polymeric material for artificial bones, comprising a solid or gel-form base containing, as the main component, either a polysaccharide having carboxyl groups or their derivatives, or a polysaccharide having carboxyl groups or their derivatives, introduced into it, which also has calcium ions bonded thereto, and shows excellent ability to form apatite.

## Description

### Technical Field:

The invention of the present application relates to a polymeric material for artificial bones. More specifically, the invention of the present application relates to a novel polymeric material for artificial bones which can effectively form an apatite layer similar to that of bones in a simulated body fluid.

### Background of the Invention:

It has been known that the bone is a composite with a three-dimensional structure formed exquisitely from fine crystals of apatite, an inorganic substance, deposited on fibers of collagen, an organic substance. Various approaches have been studied, so far, for finding a method to artificially build a similar structure.

However, all such methods employ aqueous solutions with very high concentrations of ions, as compared with the ion concentrations of body fluid, for forming an apatite layer. Such aqueous solutions can only form apatite layers differing greatly in structure and composition from those of bones in a living body, and the materials obtained therefrom cannot be readily joined with living bones.

Under these circumstances, we, the inventors of the present invention, have discovered the usefulness of a method imitating *in vivo* reactions (a biomimetic method), and have devoted ourselves to the search for a method to deposit apatite on an organic polymer base. However, we had not been able to find any polymeric material, so far, that could be used to effectively deposit a large amount of apatite with structure and tissue close to those of the inorganic substance in bones.

For example, we experimented with the use of cellulose base as the organic polymer, taking in consideration its hydrophilic property and the reactivity of the OH group, and tried to form an apatite layer in a simulated body fluid by introducing onto the surface of the base a silanol (Si-OH) group known for being effective in the nucleation of apatite. However, we have found that the apatite layer formation was too slow for this method to be effective.

The object of the present invention is, therefore, to overcome the above problems and to provide a novel polymeric material for artificial bones, which can more effectively form an apatite layer in a simulated body fluid, with structure and composition showing mechanical properties similar to those of bones.

### Disclosure of the Invention:

To attain the above object, provided as the first invention is a polymeric material for artificial bones, comprising a solid or gel-form base containing, as its main component, a polysaccharide containing carboxyl groups or their derivatives, or a polysaccharide having carboxyl groups or their derivatives introduced to it, and has calcium ions bonded thereto.

Provided as the second invention is a polymeric material for artificial bones, as described in Claim 1, in which the solid base is in the form of a porous solid, fiber, film, or bulk. Provided as the third invention is a polymeric material for artificial bones in which the polysaccharide is a natural polysaccharide containing carboxyl groups, or a polysaccharide derivative having carboxyl groups introduced to it.

Also, as the fourth invention, a polymeric material for artificial bones, on which an apatite layer can form in a simulated body fluid, is provided, and as the fifth invention, a method of manufacturing a polymeric material for artificial bones according to the inventions of Claims 1, 2, or 3, comprising the contact of a solid or gel-form base containing, as its main component, a polysaccharide containing carboxyl groups or their derivatives, or a polysaccharide having carboxyl groups or their derivatives introduced into it, with a solution containing calcium ions, to crosslink the said carboxyl groups or their derivatives. Furthermore, as the sixth invention, an artificial bone structure comprising a polymeric material for artificial bones according to the invention of Claim 1, 2, 3, or 4, with an apatite layer formed on its surface, is provided.

### Brief Description of the Drawings:

Figure 1 is a diagram showing a process that exemplifies the present invention, with the results of apatite formation.
Figure 2 is a drawing submitted for the SEM and SEM-EDX photographs of sample L, exemplifying the present invention. (1) shows a CMC gel, (2) shows the gel of (1) soaked in 1.0 SBF for two weeks, and (3) is a drawing submitted for the SEM-EDX photograph of (2) which shows it in lower magnification.
Figure 3 is a drawing submitted for the SEM and SEM-EDX photographs of sample N, exemplifying the present invention. (4) shows a CMC gel, (5) shows the gel of (4) after TEOS treatment, (6) shows (5) immersed in 1.0 SBF for two weeks, and (7) is a drawing based on the SEM-EDX photograph of (6) which shows it in lower magnification.

### Best Mode for Carrying Out the Invention:

The invention of the present application indicated above is described in further detail.

The polymeric material for artificial bones according to the invention of the present application contains, as stated before, a polysaccharide solid or gel-form base as its main component, and has calcium ions bonded thereto, where the polysaccharide composing the polymeric material has carboxyl groups or their derivatives, or has such groups introduced to it. Such derivatives are defined as various groups obtained by replacing the hydrogen atom of carboxyl groups (-COOH) to give, for example, -COOR (ester) or -COOM (a salt in which M is a metal atom), which do not hinder the bonding of calcium ions (Ca²⁺), but rather contribute to it. Examples include esters or salts which can readily form -CO-O- in the presence of water. They may also be a type of derivative which works as a protective group capable of forming -CO-O- when calcium ions are bonded to the polysaccharide.

The polysaccharide may contain carboxyl groups or their derivatives within its own molecular structure, or have such groups introduced therein by chemical reactions.

The polysaccharide which is the main component of the polymeric material for artificial bones according to the present invention may be any natural or synthetic substance, but is preferably a cellulose-type or chitin-type polysaccharide, due to their ease in obtaining and handling, and their high structural strength and stability. It is needless to say that other polysaccharides may also be used.

Specific examples of the polysaccharides include carboxymethyl cellulose (CMC), carboxylcellulose and carboxymethyl chitin.

The solid base for the polymeric material for artificial bones may be in any of the various forms available, including fibers, bulk and film.

The bonding of calcium ions to the solid or gel-form base may be carried out by, for example, allowing it to come into contact with a solution containing calcium ions. Such contact may be carried out by soaking the solid or gel-form base in an aqueous solution of calcium ions (Ca²⁺), or more specifically of Ca(OH)₂.

The bonding of calcium ions to the base is first considered as the bonding of calcium ions with the carboxyl groups. In other words, a divalent calcium ion combines with two univalent carboxyl groups, thereby functioning as a crosslinking group for the two carboxyl groups. Therefore, it is desirable that the bonding of calcium ions be carried out as close to saturation as possible. The physical adsorption of calcium ions to the polymer material for artificial bones is also taken into consideration.

The polymeric material for artificial bones according to the present invention forms apatite with structure and composition similar to those of bones on its surface, when coming in contact with human body fluid. The formation of the apatite layers is far more efficient than formerly known methods. According to the present invention, a method for forming an artificial bone structure, as described above, is also provided.

Thus provided, according to the present invention, is a polymeric material for artificial bones, on which an apatite layer is formed in a simulated body fluid. An example of such simulated body fluid is SBF [T. Kokubo, H. Kushitani, S. Sakka, T. Kitsugi and T. Yamamuro: "Solutions able to reproduce in vivo surface-structure changes in bioactive glass-ceramic A-W", J. Biomed. Mater., Res. 24, 721-734 (1996)]. SBF is an aqueous solution with ion concentrations approximately equal to those of human body fluid, as shown below.

**Table 1**

| | Concentration of ions (in mM) |
|---|---|
| Na⁺ | 142 |
| K⁺ | 5.0 |
| Mg²⁺ | 1.5 |
| Ca²⁺ | 2.5 |
| Cl⁻ | 148 |
| HCO₃⁻ | 4.2 |
| HPO₄²⁻ | 1.0 |
| SO₄²⁻ | 0.5 |

The polymeric material for artificial bones according to the present invention as described so far forms hydroxyapatite, Ca₁₀(PO₂)₆(OH)₂, in a simulated body fluid, and can be used as artificial bones in various areas of the body, such as artificial vertebrae, artificial intervertebral disk, artificial ilium, artificial neck bone, and artificial skull.

The invention will now be described in further detail based on the following examples.

### Examples:

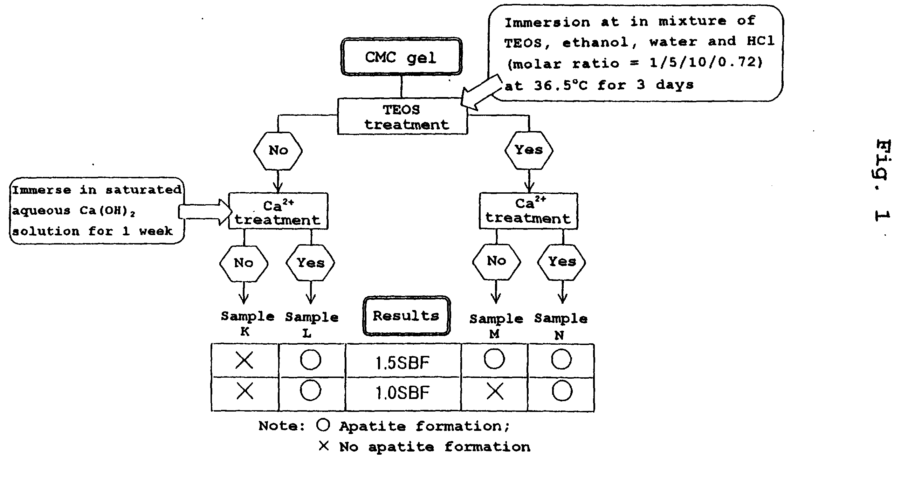

A commercially available product of CMC (carboxymethyl cellulose) was chosen as cellulose having -COOH and -COONa groups (D.S. = 0.7), and was gelated according to literature: U. Anbergen and W. Oppormann, Polymer, 31, 1854 (1990). The gel was treated through various processes as shown in Figure 1, and the process of apatite formation in 1.0 SBF and 1.5 SBF were evaluated by SEM (scanning electron microscope) and energy dispersion X-ray spectroscopy (EOX).

Here, SBF is an aqueous solution having ionic concentrations approximately equal to those of human body fluid, as described before.

The TEOS (tetraethoxysilane) treatment of the gel was carried out by immersing it in a mixture of TEOS, ethanol, water and 1N-HCl (molar ratio = 1/10/5/0.72), at 36.5°C for three days.

The Ca²⁺ treatment of the gel was carried out by immersing it in a saturated aqueous solution of Ca(OH)₂ for a week.

Figure 1 also shows the results of apatite formation.

From these results, the following was observed:
(i) Apatite can not nucleate with carboxyl groups alone (Sample K).
(ii) Even after TEOS treatment, without Ca²⁺ treatment, apatite can only be formed in 1.5 SBF (Sample M).
(iii) Samples of CMC treated with Ca²⁺ show apatite nucleation in 1.0 SBF, with or without TEOS treatment, and show very rapid formation of apatite(Samples L and N).

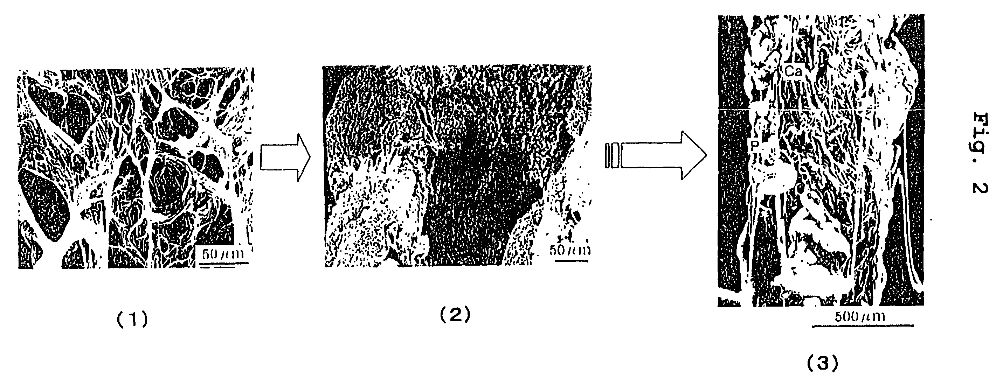

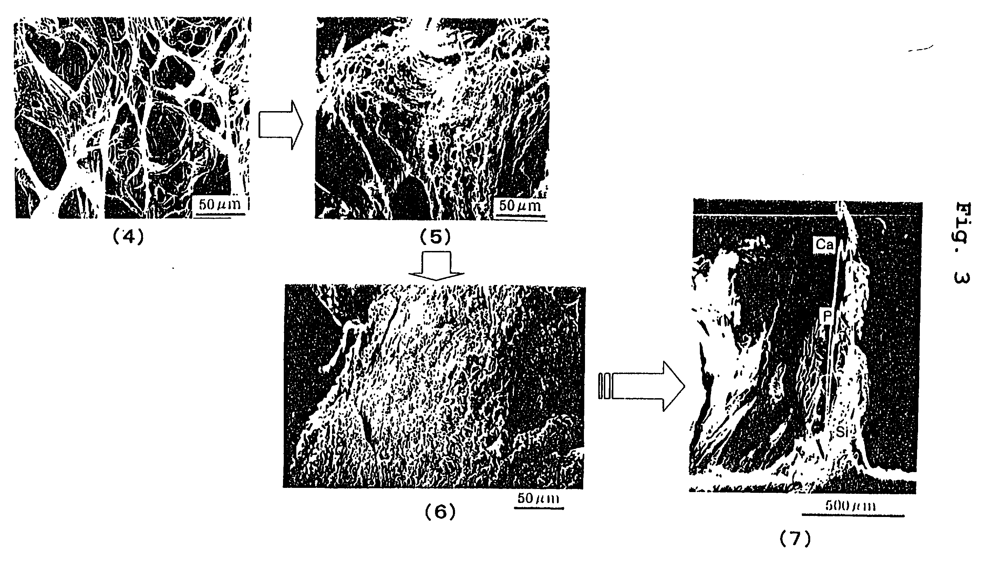

Figure 2 is a drawing based on the SEM and SEM-EDX photographs of Sample L, and Figure 3 is a drawing based on the SEM and SEM-EDX photographs of Sample N.

From the above results, it can be concluded that Ca²⁺ has a higher apatite nucleation ability than Si-OH groups, and that polysaccharides with carboxyl groups exhibit a high apatite nucleation ability when bonded with Ca²⁺.

### Industrial Applicability:

According to the invention of the present application, a novel polymeric material for artificial bones which can form apatite efficiently in a way similar to *in vivo* reaction, as described in detail above, is provided.

## Claims

1. A polymeric material for artificial bones, comprising a solid or gel-form base containing, as its main component, a polysaccharide containing carboxyl groups or their derivatives, or a polysaccaride having introduced therein carboxyl groups or their derivatives, and have calcium ions bonded thereto.

2. A polymeric material of claim 1, wherein the solid base is a porous solid, fiber, film, or a bulk.

3. A polymeric material of claim 1 or 2, wherein the polysaccharide is a natural polysaccharide containing carboxyl groups, or a polysaccharide derivative wherein carboxyl groups are introduced .

4. A polymeric material for artificial bones on which an apatite layer can form in a simulated body fluid.

5. A method for manufacturing a polymeric material of claims 1, 2, or 3, comprising the contact of a solid or gel-form base containing, as its main component, a polysaccharide containing carboxyl groups or their derivatives, or a polysaccharide having introduced therein carboxyl groups or their derivatives, with a solution containing calcium ions, wherein said groups are crosslinked by calcium ions.

6. An artificial bone structure formed by a polymeric material of claims 1, 2, 3, or 4, wherein an apatite layer is formed on the surface of the said material.
